# EUROPÄISCHE PATENTSCHRIFT

(11) **EP 3 243 495 B1**
(45) Veröffentlichungstag und Bekanntmachung des Hinweises auf die Patenterteilung: **08.09.2021**
(21) Anmeldenummer: 17170325.9
(22) Anmeldetag: 10.05.2017
(51) Int. Cl.: A61H 1/02

(54) **VORRICHTUNG ZUR ZEITWEISEN STRECKUNG UND ENTLASTUNG DER WIRBELSÄULE DES MENSCHLICHEN KÖRPERS**
DEVICE FOR THE TEMPORARY STRETCHING AND RELIEVING OF THE SPINE OF A HUMAN BEING
DISPOSITIF D'ALLONGEMENT PÉRIODIQUE ET SOULAGEMENT DE LA COLONNE VERTÉBRALE DU CORPS HUMAIN

(30) Priorität: 10.05.2016 DE 202016003031 U; 01.07.2016 DE 202016004118 U
(43) Veröffentlichungstag der Anmeldung: 15.11.2017
(73) Patentinhaber: Kienle-Holzverarbeitungs GmbH, 86879 Wiedergeltingen (DE)
(72) Erfinder: KIENLE, Josef, 86879 Wiedergeltingen (DE)
(74) Vertreter: Kruspig, Volkmar

(56) Entgegenhaltungen:
- DE-A1- 2 113 385
- DE-A1-102004 035 173
- DE-U1- 20 300 393
- JP-A- H1 033 632
- US-A- 3 890 963
- US-B1- 7 097 628

## Beschreibung

Die Erfindung betrifft eine Vorrichtung zur zeitweisen Streckung und Entlastung der Wirbelsäule des menschlichen Körpers, bestehend aus einer entlang einer Schiene motorisch verfahrbaren Einheit, diese umfassend eine Vielzahl von Gurten oder Riemen zum ventral lösbaren Fixieren des Oberkörpers im Bereich unterhalb der Schultern, wobei der Oberkörper dorsal an der verfahrbaren Einheit zur Anlage kommt, gemäß Oberbegriff des Anspruchs 1.

Aus der GB 2 359 751 A ist eine Traktionsvorrichtung in Form eines Tisches bekannt, welcher zwei zueinander relativ verschiebbare Einheiten aufweist. Der zu behandelnde Patient wird mittels Gurt auf dem Tisch fixiert und kann manuell durch Verschiebung der Tischplatten zueinander eine gewisse Traktion der Wirbelsäule herbeiführen.

Die US 2014/0214044 A1 zeigt eine transportable Vorrichtung zur Justierung der Wirbelsäule, wobei der Patient mit dem Oberkörper in ein Gurtsystem eingespannt wird. Mittels manueller Handhabe kann eine Traktion der Wirbelsäule vorgenommen werden.

Die US 4,608,969 betrifft eine transportable Traktionsvorrichtung, die einen beweglichen Schlitten auf einer Schiene aufweist, wobei der Schlitten über ein Gurtsystem verfügt, welches den Brustkorb des Patienten fixiert. Mittels manuell betätigbarer Vorspanneinrichtung erfolgt dann die gewünschte Wirbelsäulenentspannung bzw. Traktion.

Die US 2015/0202111 A1 offenbart eine transportable Vorrichtung zur mechanischen Traktion der Wirbelsäule.

Bei der US 5,462,518 wird der Brustkorb eines Patienten mittels Gurt in einer Traktionsvorrichtung arretiert. Die Vorrichtung weist darüber hinaus eine Rohranordnung auf, an welcher ein jeweils gewünschter Traktionsgrad vorjustierbar ist. Durch Armbewegungen des Probanden erfolgt die angestrebte Wirbelsäulentraktion.

Aus der US 6,007,568 ist ein Tisch zur Ausführung von chiropraktischen Schritten an einem Patienten vorbekannt, wobei der Tisch bewegliche Abschnitte und einen fest verankerten weiteren Abschnitt aufweist. Die beweglichen Abschnitte befinden sich im Hals- und Brustabschnitt des Patienten. Selbiger wird mittels Gurten am bzw. auf dem Tisch fixiert. Je nach Wirbelsäulenfehlstellung kann der Patient über die beweglichen Abschnitte des Tisches ausgerichtet werden.

Aus der US 7 097 628 B1 ist eine Vorrichtung zur zeitweisen Streckung und Entlastung der Wirbelsäule vorbekannt, wobei diese aus einer entlang einer Schienenanordnung motorisch verfahrbaren Einheit besteht und eine Vielzahl von Gurten zum ventral lösbaren Fixieren des Oberkörpers im Bereich unterhalb der Schulter besitzt.

Die DE 10 2004 035 173 A1 offenbart eine Extensionsvorrichtung, die aus einer Liegefläche besteht. Die Liegefläche soll über eine Schiene verschiebbar sein, jedoch in ihrer waagerechten Position verbleiben. Über die Liegefläche ist ein Wärme- und/oder Magnetfeld und/oder eine Vibration auf einer darauf liegenden Person aufbringbar.

Aus der WO 2003/039659 A1 ist ein Massagegürtel bekannt, wobei der Gürtel eine Kombination aus Wärmetherapie sowie Massage mittels oszillierender Einheit ermöglicht.

Die gattungsbildende EP 2 764 853 A1 betrifft eine maschinelle Vorrichtung zur Wirbelsäulenkorrektur. An einem ständerartigen Gestell ist eine Vielzahl von Riemen befindlich, um den Oberkörper eines Menschen zu fixieren. An der Säule wiederum ist ein verstellbarer, beweglicher Sitz fixiert. Der Sitz führt bezogen auf die Riemen zum Fixieren des menschlichen Oberkörpers eine Relativbewegung aus. Der Sitz kann insofern angehoben und abgesenkt werden. Mit dem Absenken des Sitzes wird eine Entlastung der Wirbelsäule bewirkt und insofern eine Therapiemaßnahme realisiert.

Den bekannten Vorrichtungen zur Entlastung der Wirbelsäule ist der Nachteil gemeinsam, dass diese einen relativ hohen apparativen Aufwand erfordern und für den Heimgebrauch nicht oder nur sehr bedingt geeignet sind. Insbesondere ist bei den bekannten technischen Lösungen keine Möglichkeit gegeben, selbige in ein einfaches Sitzmöbel, z.B. einen Polstersessel zu integrieren oder ein derartiges Sitzmöbel mit einer Behandlungsvorrichtung zu komplettieren. Darüber hinaus genügt eine zeitweise Entlastung im Sinne einer Streckung der Wirbelsäule nicht den Anforderungen an ein positives chiropraktisches Ergebnis, so dass sich insbesondere für den privaten Gebrauch diesbezügliche technische Geräte nicht durchsetzen konnten.

Aus dem Vorgenannten ist es daher Aufgabe der Erfindung, eine weiterentwickelte Vorrichtung zur zeitweisen Streckung und Entlastung der Wirbelsäule des menschlichen Körpers anzugeben, die einfach bedienbar ist und welche das Wohlbefinden des Patienten in jeder Form fördert. Darüber hinaus soll die Vorrichtung zur Nachrüstung gängiger Sitzmöbel oder aber auch zur Integration in derartige Sitzmöbel geeignet sein.

Die Lösung der Aufgabe der Erfindung erfolgt durch eine Vorrichtung gemäß der Merkmalskombination nach Anspruch 1, wobei die Unteransprüche zweckmäßige Ausgestaltungen und Weiterbildungen umfassen.

Es wird demnach von einer Vorrichtung zur zeitweisen Streckung und Entlastung der Wirbelsäule des menschlichen Körpers ausgegangen.

Die Vorrichtung besteht aus einer entlang einer Schiene oder Säule motorisch verfahrbaren Einheit. Die motorisch verfahrbare Einheit umfasst eine Vielzahl von Gurten oder Riemen oder ähnlichen Befestigungsmitteln zum ventral lösbaren Fixieren des Oberkörpers des Probanden, und zwar im Bereich unterhalb der Schultern. Mit dem Fixieren kommt der Oberkörper dorsal an der verfahrbaren Einheit zur Anlage.

Erfindungsgemäß ist darüber hinaus im Bereich der Lendenwirbel an der Schiene eine mit einer Schwingungserzeugungseinrichtung in Verbindung stehende, dorsal orientierte Massageplatte angeordnet.

Der besondere Vorteil bei Nutzung der erfindungsgemäßen Vorrichtung besteht nun darin, dass es einerseits zu einer angenehmen, in Stufen oder stufenlos vollzogenen Streckung der Wirbelsäule des Patienten kommt. Darüber hinaus erzeugt die dorsal orientierte Massageplatte im Bereich der Lendenwirbel und der dortigen umgebenden Muskulatur ein wohltuendes, einer Wärmeempfindung gleichkommendes Entspannungsgefühl. Vorteilhaft ist die Massageplatte mit einer konvexen Wölbung versehen, um den anatomischen Anforderungen (der Wirbelsäule) besser zu genügen.

Erfindungsgemäß ist auch die Massageplatte in ihrer Lage verstellbar. Diese Verstellung kann höhenmäßig, aber auch seitlich erfolgen.

Die Steuerung der Streckung der Wirbelsäule mittels motorisch verfahrbarer Einheit kann im Sinne eines Anhebens und Absenkens in verschiedenen Zyklen mit Haltepositionen realisiert werden.

Gemäß der Erfindung ist die verfahrbare Einheit lehnenartig ausgeführt und in einer doppelten Schiene beweglich gelagert. Die doppelte Schiene kann durch Beabstandung zweier Schienen realisiert sein, wobei die Führung über eine an sich bekannte Nut-Feder-Paarung oder dergleichen Mittel realisierbar ist.

Der Antrieb ist beispielsweise mittels einer rotierenden Welle unter Nutzung eines Schneckengewindes ausführbar.

In einer bevorzugten Weiterbildung der Erfindung befindet sich im Abstandsraum zwischen den vorerwähnten Schienen die elektrische Antriebseinheit, so dass die Vorrichtung möglichst kompakte Abmessungen aufweist.

Dorsal sind die Schienen mit einem Bügel zum Befestigen der Vorrichtung an einem Sitzmöbel weiterbildbar. Mit Hilfe dieser Bügel kann die Vorrichtung über die Lehne eines an sich bekannten Sitzmöbels gehangen und dort befestigt werden. Zur Schonung der Lehne ist eine Stützfläche ausgebildet, welche auftretende Kräfte auf den Sitz des Möbels ableitet. In Weiterbildung der Erfindung kommt eine höhenverstellbare Wandhalterung für die Vorrichtung zum Einsatz.

In einer wiederum bevorzugten Ausführungsform der Erfindung führt die Massageplatte eine oszillierende Bewegung in dorsaler Ebene aus. Maßgeblich sind hier angenehme Vibrationen und Schwingungen, die zur gewünschten Lockerung der die Wirbel umgebenden Muskulatur mit Ausbildung eines Wärmegefühls führen.

Alternativ zur oszillierenden Bewegung der Massageplatte besteht die Möglichkeit, dass ein Körperschallwandler eingesetzt wird, welcher Schwingungen erzeugt, die auf die Massageplatte übertragen werden. Diese Schwingungen, die zu einer gezielten Vibration der Massageplatte bzw. deren Oberfläche führen, ergeben bei sinnvoller Anwendung ein Auflockern der Rückenmuskulatur. In einem solchen Fall wird ein Vibrationsmodul gebildet.

Dieser Vibrationsmodul ist durch eine Massageplatte mit oszillierender Bewegung austauschbar.

Ergänzend besteht die Möglichkeit, sowohl ein Vibrationsmodul als auch ein Massagemodul baulich zu vereinigen und die entsprechenden Module wahlweise zu aktivieren, je nach gewünschtem Behandlungsziel oder Wohlbefinden des Patienten.

In Weiterbildung der Erfindung besteht die Möglichkeit, alle wesentlichen Funktionen durch eine Fernbedienung zu steuern, so dass durch den Patienten der Streckungsgrad, die Massage oder Vibrationsstärke und die Behandlungsdauer vorgeb- und wählbar ist und auch jederzeit die Behandlung unterbrochen werden kann. Die Fernbedienung kann dabei über einen integrierten Timer verfügen, um ein automatisches Abschalten der erfindungsgemäßen Vorrichtung nebst Zurückfahren in die Ausgangsposition zu veranlassen.

Die Schiene kann gemäß Ausgestaltung der Erfindung in eine kaudale Sitzfläche übergehen oder mit einer solchen Sitzfläche verbunden sein.

Bevorzugt stützt sich die verfahrbare Einheit jedoch über den Bügel zum Befestigen der Vorrichtung an einem Sitzmöbel oder unmittelbar durch Befestigung am Sitzmöbel ab.

Sowohl die motorisch verfahrbare Einheit als auch die Schwingungserzeugungseinrichtung verfügen über eine separate Ansteuereinheit zur individuellen Betätigung.

Diese Ansteuereinheit bzw. die Ansteuereinheiten können frei programmierbar ausgebildet sein. Über eine Betätigungseinheit können Befehle und/oder Programme für die motorischen Antriebe eingegeben bzw. entsprechende Aktivitäten ausgelöst werden. Die zum Betreiben der Vorrichtung erforderlichen steuerungsseitigen Aspekte und Programme können so realisiert werden, dass neben einer Zeitsteuerung auch eine maximale Traktionsgeschwindigkeit und Fahrwegbegrenzung vorgesehen ist.

Sowohl die Gurte oder Riemen als auch die zum menschlichen Körper gerichtete Oberflächenseite der Massageplatte verfügen über eine Abpolsterung. Diese Abpolsterung kann aus hygienischen Gründen abnehmbar bzw. austauschbar ausgeführt sein. Im Bereich des Untergreifens der Achseln des Probanden ist weiterhin für eine ausreichende Abpolsterung der dort befindlichen Riemen oder Gurte gesorgt, so dass insgesamt auch bei einer länger andauernden Behandlung für den Probanden keine unangenehmen Druckbelastungen resultieren.

In einer Ausgestaltung der Erfindung weist die verfahrbare Einheit eine Aufnahme zum Befestigen einer anatomisch auswählbaren Gruppe von Gurten oder Riemen auf. Diese auswählbare Gruppe kann an einer Trägerplatte fixiert sein, so dass je nach zu behandelnder Person eine leichte Austauschbarkeit ohne aufwendige Umbaumaßnahmen gewährleistet wird.

Steuerungsseitig besteht die Möglichkeit, mit dem Erreichen eines vorgegebenen Extraktionswerts der Wirbelsäule durch kraniale Bewegung der verfahrbaren Einheit die Schwingungserzeugungseinrichtung zuzuschalten, so dass selbige im Entspannungszustand der Wirbel aktiv ist.

Die Erfindung soll nachstehend anhand eines Ausführungsbeispiels sowie unter Zuhilfenahme von Figuren näher erläutert werden.

Hierbei zeigen:
- Fig. 1: eine Darstellung der erfindungsgemäßen Vorrichtung, einsetzbar in ein sesselartiges Sitzmöbel im Zustand der erreichten unteren Position der verfahrbaren Einheit;
- Fig. 2: eine Darstellung vergleichbar mit derjenigen nach Fig. 1, jedoch mit einem Zustand des Verfahrens zum Zweck der Extraktion der Wirbelsäule in Richtung nach oben, d.h. bei einem im Sessel sitzenden Patienten in Richtung kranial; und
- Fig. 3: eine Weiterbildung der Vorrichtung mit gewölbter Oberfläche der Massageplatte und Fernbedienung.

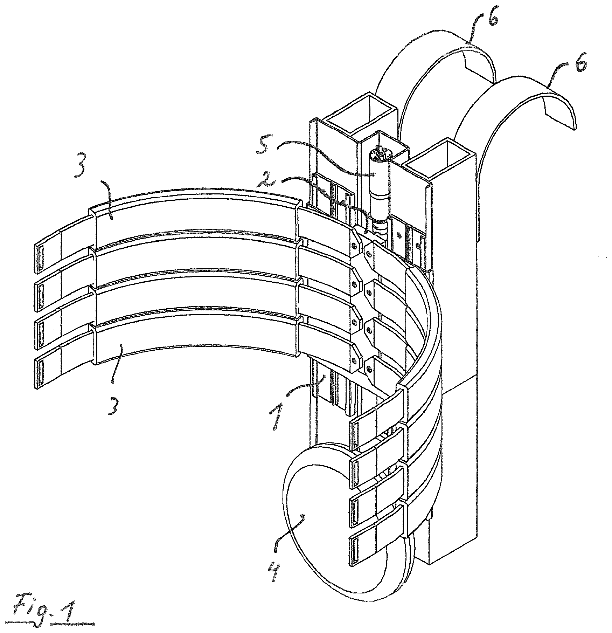

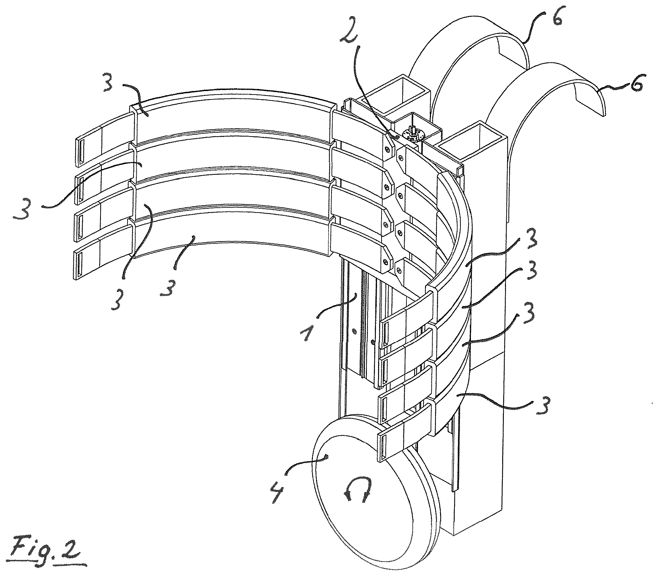

Den Darstellungen nach den Fig. 1 und 2 ist gemeinsam die Ausbildung einer entlang einer Schienenanordnung 1 motorisch verfahrbaren Einheit in Form einer flächigen Platte 2, die wiederum einen Träger fixiert, welcher Gurtpaare 3 trägt.

Mindestens das obere Gurtpaar ist mit einer Abpolsterung versehen.

Am unteren Ende ist die im gezeigten Beispiel vorhandene doppelte Schienenanordnung mit einer im Bereich der Lendenwirbel an der Schiene angreifenden Schwingungserzeugungseinrichtung in Verbindung stehend. Die Schwingungserzeugungseinrichtung nimmt eine dorsal orientiere Vibrations- oder Massageplatte 4 auf, die gemäß den Darstellungen ebenfalls oberflächenseitig eine Abpolsterung in Form eines Schaumstoffkissens oder dergleichen besitzt.

Die verfahrbare Einheit ist quasi lehnenartig ausgeführt und wird über die doppelte Schienenanordnung beweglich gelagert.

Zwischen der doppelten Schienenanordnung befindet sich ein Elektromotor 5, welcher mit einer Schneckenwelle in Verbindung steht, die wiederum in eine Spindelmutter (nicht gezeigt) eingreift, welche mit der verfahrbaren Einheit zusammenwirkt. Rückseitig weisen die Schienen einen Bügel 6 zum Befestigen der Vorrichtung am Sitzmöbel gemäß den figürlichen Darstellungen auf.

Die im unteren Bereich der Vorrichtung erkennbare Massageplatte 4 führt z.B. eine oszillierende, insbesondere schwingende und hin- und hergehende Bewegung (Pfeildarstellung) in der dorsalen Ebene aus. Alternativ vibriert die Oberfläche der Massageplatte. Wahlweise kann der Nutzer zwischen Vibration und Massage wählen.

Im Benutzungsfall nimmt der Patient im Sitzmöbel Platz und führt den Rücken an die verfahrbare Einheit mit Gurten. Nach straffem Anlegen der Gurte bleibt der Rückenkontakt mit der Vorrichtung und insbesondere mit der Massageplatte erhalten. Über eine nicht dargestellte Bedieneinheit beginnt nun der Vorgang des Verfahrens der Einheit mit Gurt oder Riemen in Richtung kranial, d.h. entsprechend den figürlichen Darstellungen nach oben. Hierbei untergreifen und umgreifen die Gurte den menschlichen Körper und bewegen diesen aus einer quasi erschlafften Position heraus mit der Folge der Entspannung und Extraktion der Wirbel. Zusätzlich beginnt der Massagevorgang unter Nutzung der Schwingungserzeugungseinrichtung mit Massageplatte.

Der Vorteil der gezeigten Vorrichtung besteht darin, dass diese in bekannte Sitzmöbel integrierbar, aber auch leicht nachrüstbar ausgeführt ist. Eine diesbezügliche Wohlfühlbehandlung kann also auch ohne weiteres im häuslichen Bereich ohne ärztliche Aufsicht vorgenommen werden.

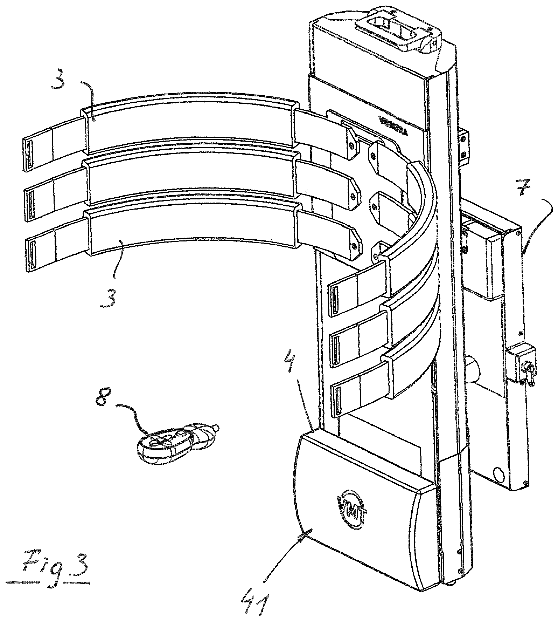

Die Verfahrbewegung erfolgt sehr schonend und langsam, d.h. keinesfalls ruckartig. Der Patient oder Proband ist jederzeit in der Lage, über die nicht gezeigte Steuerungseinheit oder eine Fernbedienung 7 (siehe Figur 3) eine Umkehrbewegung der verfahrbaren Einheit auszulösen, ein Verharren an einer bestimmten Position vorzugeben und/oder die Behandlung zu beenden bzw. zu unterbrechen. Gleiches gilt für die Ansteuerung der Schwingungsgrößen für die Massageplatte.

Die Figur 3 zeigt eine Weiterbildung der erfindungsgemäßen Vorrichtung mit einer gewölbten Oberfläche 41 der Massageplatte 4 und einer angedeuteten Fernbedienung 8.

Die Wölbung der Massageplatte 4 ist hierbei konvex realisiert im Sinne einer Lordosenabstützung. Diese anatomisch optimierte Wölbung verbessert den Behandlungserfolg und führt zu einem gesteigerten Wohlbefinden der Patienten während der Behandlung.

Die Massageplatte 4 kann einerseits Körperschall, das heißt Vibrationen auf den Patienten übertragen, wobei diesbezüglich im Innen der Massageplatte eine Körperschall erzeugende Einrichtung ausgebildet ist.

Andererseits kann die bereits erläuterte Massagefunktion wirksam werden. Diesbezüglich ist denkbar, im Inneren der Massageplatte eine Exzenterwelle vorzusehen, welche massageähnliche Bewegungen auf die Oberfläche der Massageplatte überträgt.

Die Massage bzw. Vibrationsvorrichtung ist bei einer Weiterbildung der Erfindung höhenverstellbar. Dies kann händisch aber auch elektromotorisch erfolgen. Auf diese Weise ist es möglich, eine Massage gezielt im Bereich der Lendenwirbelsäule durchzuführen.

In den Figuren 1 und 2 ist angedeutet, dass mittels der Bügel 6 die Vorrichtung an einem Sitzmöbel befestigbar ist.

Zur Verbesserung der Kraftabtragung und zur Schonung der Lehne besteht die Möglichkeit, die Auflage bezüglich des Sessels zu vergrößern und insofern die Sitzfläche des Sitzmöbels zu nutzen, um das Gewicht und die auftretenden Kräfte abzutragen.

Alternativ besteht die Möglichkeit, das Gerät an einer Wandhalterung 7 zu befestigen bzw. einzuhängen, wobei die Wandhalterung höhenverstellbar ist.

Die Möglichkeit des Befestigens der Vorrichtung an einer Wandhalterung 7 ist durch die Darstellung gemäß Figur 3 angedeutet.

## Patentansprüche

1. Vorrichtung zur zeitweisen Streckung und Entlastung der Wirbelsäule des menschlichen Körpers, bestehend aus einer entlang einer Schienenanordnung (1) motorisch verfahrbaren Einheit, diese umfassend eine Vielzahl von Gurten oder Riemen (3) zum ventral lösbaren Fixieren des Oberkörpers im Bereich unterhalb der Schultern, wobei der Oberkörper dorsal an der verfahrbaren Einheit zur Anlage kommt,
**dadurch gekennzeichnet, dass**
im Bereich der Lendenwirbel bei der Benutzung an der Schienenanordnung (1) eine mit einer Schwingungserzeugungseinrichtung in Verbindung stehende, dorsal orientierte, in ihrer Lage verstellbare Vibrations- oder Massageplatte (4) angeordnet ist, wobei die verfahrbare Einheit lehnenartig ausgeführt in einer doppelten Schiene beweglich gelagert ist, wobei im Abstandsraum zwischen den Schienen eine elektrische Antriebseinheit ausgebildet ist und dorsal die Schienen mit einem Bügel (6) zum Befestigen der Vorrichtung an einem Sitzmöbel versehen sind.

2. Vorrichtung nach Anspruch 1,
**dadurch gekennzeichnet, dass**
die Massageplatte (4) eine oszillierende Bewegung in dorsaler Ebene ausführt.

3. Vorrichtung nach einem der vorangegangenen Ansprüche,
**dadurch gekennzeichnet, dass**
die Schiene (1) in eine kaudale Sitzfläche übergeht oder mit einer solchen Sitzfläche verbunden ist.

4. Vorrichtung nach einem der vorangegangenen Ansprüche,
**dadurch gekennzeichnet, dass**
die motorisch verfahrbare Einheit und die Schwingungserzeugungseinrichtung über eine separate Ansteuereinheit zur individuellen Betätigung verfügen.

5. Vorrichtung nach Anspruch 4,
**dadurch gekennzeichnet, dass**
die Ansteuereinheiten programmierbar ausgebildet sind.

6. Vorrichtung nach einem der vorangegangenen Ansprüche,
**dadurch gekennzeichnet, dass**
sowohl die Gurte oder Riemen (3) als auch die zum menschlichen Körper gerichtete Oberflächenseite der Massageplatte (4) über eine Abpolsterung verfügen.

7. Vorrichtung nach einem der vorangegangenen Ansprüche,
**dadurch gekennzeichnet, dass**
die verfahrbare Einheit eine Aufnahme zum Befestigen einer anatomisch auswählbaren Gruppe von Gurten oder Riemen (3) aufweist.

8. Vorrichtung nach einem der vorangegangenen Ansprüche,
**dadurch gekennzeichnet, dass**
mit dem Erreichen eines vorgegebenen Extraktionswerts der Wirbelsäule durch kraniales Bewegen der verfahrbaren Einheit die Schwingungserzeugungseinrichtung zuschaltbar ist.

9. Vorrichtung nach einem der vorangegangenen Ansprüche,
**dadurch gekennzeichnet, dass**
die zum menschlichen Körper gerichtete Oberflächenseite (41) der Vibrations-oder Massageplatte (4) eine konvexe, anatomisch angepasste Form aufweist.

10. Vorrichtung nach einem der Ansprüche 1 bis 9,
**dadurch gekennzeichnet, dass**
sowohl die elektrische Antriebseinheit als auch die Schwingungserzeugungseinrichtung funktionsseitig fernbedienbar sind.

## Claims

1. A device for the temporary stretching and relieving of the spine of a human body, composed of a unit displaceable motorically along a rail arrangement (1), which unit comprises a plurality of belts or straps (3) for ventrally fixing in a detachably manner the upper body area below the shoulders, wherein the upper body dorsally gets to rest against the displaceable unit,
**characterized in that**,
when the rail arrangement (1) is used, a positionally adjustable vibration or massage plate (4) that is dorsally oriented and in connection with a vibration generation device, is arranged in the area of the lumbar vertebrae, wherein the displaceable unit, configured in the manner of a backrest, is supported to be movable within a double rail, wherein within the distance space between the rails, an electric drive unit is formed, and the rails are dorsally provided with a bracket (6) for attaching the device on a seating furniture.

2. The device according to claim 1,
**characterized in that**
the massage plate (4) executes an oscillating movement in the dorsal plane.

3. The device according to any one of the preceding claims,
**characterized in that**
the rail (1) passes into a caudal seating surface or is connected to such a seating surface.

4. The device according to any one of the preceding claims,
**characterized in that**
the motorically displaceable unit and the vibration generation device have a separate control unit for individual actuation.

5. The device according to claim 4,
**characterized in that**
the control units are formed to be programmable.

6. The device according to any one of the preceding claims,
**characterized in that**
both the belts or straps (3) and the upper surface side of the massage plate (4) directed towards the human body have a padding.

7. The device according to any one of the preceding claims,
**characterized in that**
the displaceable unit includes a mounting device for attaching an anatomically selectable set of belts or straps (3).

8. The device according to any one of the preceding claims,
**characterized in that**,
when a predefined extraction value of the spine is reached, the vibration generation device is activable by a cranial movement of the displaceable unit.

9. The device according to any one of the preceding claims,
**characterized in that**
the upper surface side (41) of the vibration or massage plate (4) directed towards the human body includes a convex, anatomically adapted shape.

10. The device according to any one of claims 1 to 9,
**characterized in that**
both the electric drive unit and the vibration generation device are functionally remotely controllable.

## Revendications

1. Dispositif pour l'étirement et le soulagement temporaires de la colonne vertébrale du corps humain, constitué d'une unité déplaçable par voie motrice le long d'un ensemble de rails (1), ladite unité comprenant une pluralité de ceintures ou de sangles (3) pour la fixation ventrale amovible du torse corps dans la région située sous les épaules, le torse venant s'appuyer dorsalement contre l'unité déplaçable,
**caractérisé en ce que**
dans la région des vertèbres lombaires, pendant l'utilisation, une plaque de vibration ou de massage (4) reliée à un dispositif générateur de vibrations, orientée dorsalement et réglable quant à sa position est disposée sur l'ensemble de rails (1), l'unité déplaçable étant conçue à la manière d'un dossier et étant montée de façon mobile dans un double rail, une unité d'entraînement électrique étant réalisée dans l'espacement entre les rails, et les rails étant pourvus dorsalement d'un étrier (6) pour fixer le dispositif à un siège.

2. Dispositif selon la revendication 1,
**caractérisé en ce que**
la plaque de massage (4) effectue un mouvement oscillant dans un plan dorsal.

3. Dispositif selon l'une des revendications précédentes,
**caractérisé en ce que**
le rail (1) se transforme en une surface d'assise caudale ou est relié à une telle surface d'assise.

4. Dispositif selon l'une des revendications précédentes,
**caractérisé en ce que**
l'unité déplaçable par voie motrice et le dispositif générateur de vibrations disposent d'une unité de pilotage séparée pour un actionnement individuel.

5. Dispositif selon la revendication 4,
**caractérisé en ce que**
les unités de pilotage sont conçues de manière programmable.

6. Dispositif selon l'une des revendications précédentes,
**caractérisé en ce que**
aussi bien les ceintures ou sangles (3) que la face superficielle de la plaque de massage (4) tournée vers le corps humain disposent d'un rembourrage.

7. Dispositif selon l'une des revendications précédentes,
**caractérisé en ce que**
l'unité déplaçable comprend un logement pour fixer un groupe de ceintures ou de sangles (3) sélectionnable sur le plan anatomique.

8. Dispositif selon l'une des revendications précédentes,
**caractérisé en ce que**
le dispositif générateur de vibrations peut être mis en marche par déplacement crânial de l'unité déplaçable, lorsqu'une valeur d'extraction prédéterminée de la colonne vertébrale est atteinte.

9. Dispositif selon l'une des revendications précédentes,
**caractérisé en ce que**
la face superficielle (41) de la plaque de vibration ou de massage (4) tournée vers le corps humain présente une forme convexe, anatomiquement adaptée.

10. Dispositif selon l'une des revendications 1 à 9,
**caractérisé en ce que**
aussi bien l'unité d'entraînement électrique que le dispositif générateur de vibrations sont fonctionnellement télécommandables.
